Europäisches Patentamt

European Patent Office

Office européen des brevets

(11) Numéro de publication : **0 528 716 A1**

# (12) DEMANDE DE BREVET EUROPEEN

(21) Numéro de dépôt : **92402237.9**

(22) Date de dépôt : **05.08.92**

(51) Int. Cl.⁵ : **C07K 13/00, A61K 37/14**

(30) Priorité : **14.08.91 FR 9110342**

(43) Date de publication de la demande :
**24.02.93 Bulletin 93/08**

(84) Etats contractants désignés :
**AT BE CH DE DK ES FR GB GR IE IT LI LU NL PT SE**

(71) Demandeur : **PASTEUR MERIEUX SERUMS ET VACCINS, Société Anonyme :**
**58 Avenue Leclerc**
**F-69348 Lyon Cédex 07 (FR)**

(72) Inventeur : **Dellacherie, Edith**
**15 rue Jean Ploussard**
**F-54220 Malzeville (FR)**
Inventeur : **Sacco, Daniel**
**11, Boulevard Charles, V**
**F-54000 Nancy (FR)**
Inventeur : **Grandgeorge, Michel**
**12 rue du Recret**
**F-69670 Vaugneray (FR)**

(74) Mandataire : **Bernasconi, Jean et al**
**CABINET LEMOINE ET BERNASCONI 13,**
**Boulevard des Batignolles**
**F-75008 Paris (FR)**

(54) **Dérivés d'hémoglobine humaine, leur procédé de préparation, produits en dérivant et utilisation de ces dérivés et produits.**

(57) L'invention concerne, à titre de composés nouveaux, les dérivés d'hémoglobine tétramérique humaine correspondant au couplage covalent d'un tétramère d'hémoglobine et d'un composé polycarboxylique ayant au moins 4 groupements -COO⁻, ledit composé polycarboxylique assurant une réticulation interne des deux dimères de l'hémoglobine et lui conférant une affinité pour l'oxygène compatible avec une utilisation in vivo du conjugué hors de la présence de l'effecteur allostérique naturel de l'hémoglobine. Parmi les composés carboxyliques, le benzène hexacarboxylate et le benzène tétracarboxylate sont particulièrement indiqués.

FIG. 2

EP 0 528 716 A1

L'invention concerne de nouveaux dérivés d'hémoglobine humaine présentant une bonne persistance intravasculaire et une affinité pour l'oxygène inférieure à celle de l'hémoglobine libre, plus précisément compatible avec une utilisation in vivo pour le transport d'oxygène en l'absence de l'effecteur allostérique naturel de l'hémoglobine (le 2,3-diphosphoglycérate, DPG).

L'invention concerne aussi un procédé de préparation de ces dérivés, les produits en dérivant et l'utilisation de ces dérivés et produits comme transporteurs d'oxygène, notamment dans le cadre des tranfusions.

D'après les demandes de brevet français n° 2 600 894 et 2 630 329, il a été montré que des polymères hydrosolubles portant des sites polyanioniques et en particulier des sites polycarboxylates, comme par exemple des sites benzène carboxylates, pouvaient être liés de manière covalente à l'hémoglobine humaine en présence d'un carbodiimide hydrosoluble et que, même lorsque la réaction était effectuée avec la forme oxy de l'hémoglobine, les conjugués résultants avaient une affinité pour l'oxygène beaucoup plus faible que la protéine native (en l'absence de DPG).

En étudiant plus particulièrement un conjugué résultant de la fixation de polyoxyéthylène monosubstitué par un site dérivé du benzène hexacarboxylate, à l'oxyhémoglobine, il a été démontré que la poche allostérique de la protéine - et en particulier la fonction $\alpha$-aminée de la valine $\beta$ N-terminale située dans cette poche- était un site privilégié pour la fixation du polymère (E. DELLACHERIE et M. LEONARD, J. Protein Chem. 1991, vol. 10, n° 1, 61-67). La présence de groupes anioniques au voisinage du site allostérique de l'hémoglobine confère à cette dernière une faible affinité pour l'oxygène ; sa taille moléculaire étant augmentée par la présence du polymère, l'hémoglobine modifiée possède également, in vivo, une persistance intravasculaire augmentée par rapport à la forme native (diffusibilité au travers des membranes biologiques très diminuée).

L'utilisation de ces conjugués polymériques présente quatre inconvénients majeurs qui sont le risque de choc anaphylactique (constaté avec les dextranes) lié à leur utilisation répétée, les problèmes d'intolérance liés aux hauts poids moléculaires (formation de multi-tétramères), la viscosité des solutions contenant ces conjugués, ce qui, notamment, limite la pénétration tissulaire, et le coût des polymères.

On a également proposé de faire réagir ensemble de l'oxyhémoglobine et de l'acide 3,5-dibromosalicylique estérifié par des acides carboxyliques tels que l'acide fumarique (T.N. Estep et al. et E. Bucci et al., International Symposium on Red Cell Substitutes, Holiday-Inn, Fisherman's Wharf, San Francisco, Californie, USA, 16-19 mai 1989 - résumés).

A. Desbois et al. avaient étudié dans J. Mol. Biol. 1975, 92 (3), 479-493, l'action de polycarboxylates ayant de 2 à 6 groupes carboxylates sur le comportement de l'hémoglobine vis-à-vis de l'oxygène. Il n'est pas question de liaison covalente mais d'une simple interaction entre le polycarboxylate et l'hémoglobine.

La déposante a trouvé de façon très surprenante que, dans certaines conditions de réaction, la fixation d'acides benzène polycarboxyliques libres, et de préférence tétra- à hexacarboxyliques, à de l'oxyhémoglobine donnait naissance à des dérivés d'hémoglobine possédant une affinité pour l'oxygène compatible avec une utilisation in vivo hors de la présence de l'effecteur naturel et incapables de se dissocier en dimères. Cette dernière particularité apporte une solution nouvelle au problème de la fuite vasculaire et rénale de l'hémoglobine, qui permet de se passer des polymères utilisés jusqu'à présent ou d'éviter des réticulations par des agents mal contrôlés.

La déposante a en outre trouvé que l'on pouvait étendre cet enseignement à de petites molécules polycarboxyliques présentant au moins 4 groupements -COO⁻. En effet, il est apparu de façon surprenante que ces molécules pouvaient réaliser un pont intramoléculaire entre les deux dimères $\alpha \beta$ de l'hémoglobine, assurant ainsi une réticulation interne de la protéine. Ce pont fait intervenir deux liaisons covalentes, si bien qu'il est nécessaire de disposer encore d'au moins 2 groupements carboxylates pour assurer un rôle d'effecteur suffisant.

L'invention a donc pour objet un dérivé d'hémoglobine tétramérique humaine provenant du couplage covalent d'un tétramère d'hémoglobine et d'un composé polycarboxylique ayant au moins 4 groupements -COO⁻, ledit composé polycarboxylique assurant une réticulation interne des deux dimères de l'hémoglobine et lui conférant une affinité pour l'oxygène compatible avec une utilisation in vivo du conjugué hors de la présence de l'effecteur allostérique naturel de l'hémoglobine, c'est-à-dire pouvant lier l'oxygène d'une façon réversible propre à permettre la libération de l'oxygène dans les tissus irrigués. Le couplage covalent peut être réalisé indifféremment en présence ou en l'absence d'oxygène, sur l'oxy- ou la désoxyhémoglobine.

De préférence, les molécules polycarboxyliques possèdent de 4 à 6 groupements -COO⁻ et sont avantageusement des molécules monocycliques saturées ou non saturées ou des molécules linéaires ramifiées ou non ramifiées de 4 à 6 atomes de carbone pour la chaîne principale.

Des molécules préférées peuvent être des benzènes polycarboxyliques, notamment hexa- ou tétracarboxyliques, ou encore un cyclohexane polycarboxylique.

L'invention a également pour objet une solution de transport d'oxygène à base d'hémoglobine tétramérique humaine, dans laquelle l'hémoglobine est constituée au moins en partie par un dérivé selon l'invention.

L'invention a encore pour objet un procédé de préparation d'un dérivé d'hémoglobine tétramérique, dans lequel on effectue le couplage covalent de la molécule d'hémoglobine tétramérique, de préférence sous forme d'oxyhémoglobine, et d'un composé polycarboxylique tel que défini ci-dessus, dans des conditions assurant la réticulation des deux dimères de la molécule d'hémoglobine. Ce couplage s'effectue de préférence en présence d'un agent favorisant les liaisons covalentes, tel que le chlorhydrate de N'-éthyl-N-(3-diméthyl-aminopropyl) carbodiimide (EDCI) ou tout autre réactif classiquement utilisé dans la synthèse peptidique.

De préférence, on effectue le couplage dans des conditions assurant un rendement de réticulation d'au moins 50 % environ, notamment supérieur ou égal à 80 % environ, et une $P_{50}$ du dérivé supérieure ou égale à 1000 Pa, notamment supérieure ou égale à 1300 Pa (les $P_{50}$ étant mesurées en tampon Tris 0,05 M, pH 7,2 et à 25°C).

Dans le cas du benzène hexacarboxylate (BHC), le couplage covalent avec l'hémoglobine peut s'effectuer avec des rapports molaires BHC/Hb et EDCI/Hb de respectivement 3 et 8 ou 5 et 6 environ. Dans le cas du benzène tétracarboxylate (BTC), les rapports molaires BTC/Hb et EDCI/Hb peuvent être respectivement de 5 et 32 environ.

Selon une particularité avantageuse de l'invention, on peut effectuer le couplage en présence de NaCl.

L'invention a encore pour objet une solution d'hémoglobine tétramérique stabilisée obtenue par ce procédé et, de préférence, comprenant plus de 50 %, avantageusement plus de 80 %, de dérivé d'hémoglobine ayant une $P_{50}$ supérieure ou égale à 1000 Pa, de préférence supérieure ou égale à 1300 Pa.

Les tétramères d'hémoglobine modifiés selon l'invention présentent un potentiel important en thérapeutique humaine comme transporteurs d'oxygène dans tous les cas où l'on veut améliorer la délivrance d'oxygène aux tissus ou à l'organisme, par exemple en cas d'hémorragies sévères (à titre de substitut sanguin) ou de résistances vasculaires diverses (à titre de transporteurs d'oxygène de plus petite taille que les globules rouges) ou encore, par exemple, en cas d'angioplastie nécessitant une dérivation avec recours à un substitut sanguin.

L'invention a donc également pour objet l'utilisation de dérivés ou solutions selon l'invention pour la fabrication d'une préparation de transport d'oxygène chez l'homme.

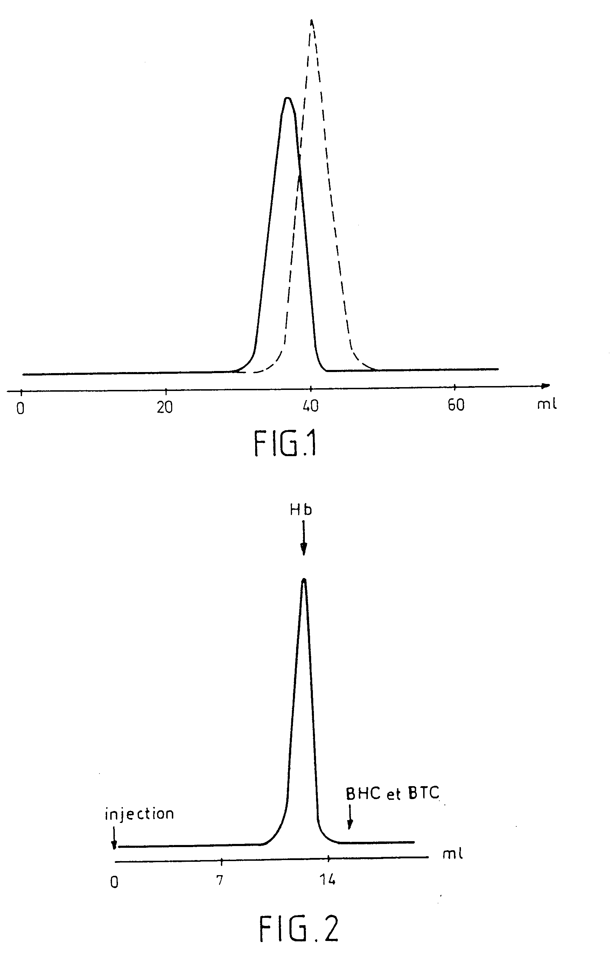

L'invention va être maintenant décrite plus en détail, en liaison avec les figures 1 à 5, à l'aide d'exemples appliqués au couplage covalent, à l'hémoglobine, de benzène hexacarboxylate (BHC) et de benzène tétracarboxylate (BTC), en présence de chlorhydrate de N'-éthyl-N-(3-diméthylaminopropyl) carbodiimide (EDCI).

La figure 1 montre la dissociation de l'hémoglobine native en dimères, mise en évidence par chromatographie d'exclusion par la taille sur gel de Sephacryl S100. Le pic en trait plein correspond au tétramère (tampon phosphate 0,05 M), et le pic en trait interrompu, qui se trouve en position retardée par rapport au tétramère, correspond aux dimères (en MgCl$_2$ 1M) ; détection à 254 nm.

La figure 2 donne le profil (chromatogramme) type obtenu en filtration sur gel (TSK G4000 SW) en milieu phosphate pour les différents dérivés d'hémoglobine obtenus conformément à l'invention. Les flèches indiquent les volumes d'élution normaux de l'hémoglobine native et des acides benzène polycarboxyliques (BHC et BTC) ; détection à 254 nm.

La figure 3 donne le profil d'élution du dérivé 4 (exemple 4, tableau I) sur colonne échangeuse de cations Mono S ; détection à 415 nm.

Les figures 4 et 5 donnent les profils des produits obtenus aux exemples 4 (tableau I) et 7 (tableau II), obtenus en filtration sur gel de Sephacryl S100 en milieu MgCl$_2$ ; détection à 254 nm.

Les techniques d'analyse des dérivés d'hémoglobine donnés en exemple sont les suivantes :

1) Chromatographie d'exclusion par la taille sur une colonne TSK G4000 SW (30 cm) (Touzart et Matignon - France) en tampon phosphate 0,05 M, pH 7,2, à 0,7 ml/mn, pour contrôler d'une part la taille de l'hémoglobine modifiée (contrôle en particulier de l'absence de réticulations intermoléculaires) et d'autre part l'absence d'acide benzène polycarboxylique résiduel.

2) Chromatographie d'échange d'ions sur colonne Mono S (25 cm) (Pharmacia - Suède) en tampon malonate 10 mM à pH 5,7, avec un gradient de LiCl de 0 à 0,3 M, pour évaluer le taux de tétramères d'hémoglobine non modifiés (débit 2,5 ml/mn).

3) Chromatographie d'exclusion par la taille sur gel de Séphacryl S100 (1,6 x 40 cm) en tampon Tris 0,1 %, pH 7,0, MgCl$_2$ 1 M, à 50 ml/h, pour évaluer le taux de dissociations en dimères de l'hémoglobine modifiée. En effet dans ces conditions, l'hémoglobine native est totalement dissociée en dimères $\alpha$ $\beta$ comme le montre la figure 1.

4) Mesure de la $P_{50}$ (pression partielle d'oxygène pour laquelle 50 % de l'hémoglobine est oxygénée) en tampon Tris 0,05 M, pH 7,2 et à 25°C.

Exemples 1 à 5 (Tableau I)

A 10 ml d'un solution d'hémoglobine humaine naturellement oxygénée, de concentration 1,4 mM, on ajoute 5,5 ml (exemples 1, 2 et 4) ou 9,25 ml (exemples 3 et 5) d'une solution aqueuse de BHC de concentration 7,8 mM préalablement amenée à pH 5,8 avec de la soude 0,1 M. Le pH de ce mélange est ajusté à 6,5 à l'aide d'HCl 0,1 M et on complète à un volume final de 30 ml avec de l'eau distillée.

On ajoute ensuite 11 mg (exemple 1), 16,5 mg (exemples 2 et 3) ou 22 mg (exemples 4 et 5) d'EDCI sous agitation. La réaction est poursuivie pendant 2 h à 20°C. On obtient ainsi les dérivés (hémoglobine réticulée) 1 à 5.

Exemple 6 (Tableau I)

On procède comme aux exemples précédents avec 10 ml de solution d'hémoglobine (1,4 mM), 5,5 ml d'une solution de BHC (7,8 mM), à pH 5,8, dans NaCl 0,1 M, et 22 mg d'EDCI.

Le pH de la solution est ajusté à 6,5 avec HCl 0,1 M et on complète à un volume final de 30 ml avec une solution de NaCl pour avoir une concentration finale de 0,1 M. On obtient le dérivé (hémoglobine réticulée) 6.

Exemples 7 à 9 (Tableau II)

A 10 ml d'une solution d'oxyhémoglobine de concentration 1,4 mM, on ajoute respectivement 4,7 ml, 9,4 ml et 18,8 ml d'une solution aqueuse de BTC de concentration 15 mM préalablement amenée à pH 5,8 avec de la soude 0,1 M. Le pH de ce mélange est ajusté à 6,5 avec HCl 0,1 M et on complète à un volume final de 30 ml avec de l'eau distillée.

On ajoute alors respectivement 22, 44 et 88 mg d'EDCI sous agitation et on laisse la réaction se poursuivre pendant 2 h à 20°C. On obtient ainsi les dérivés (hémoglobine réticulée) 7, 8 et 9.

Exemples 10 à 12 (Tableau II)

Idem exemples 7 à 9, sauf, respectivement, 18,8 ml, 4,7 ml et 4,7 ml de solution de BTC et 66, 44 et 88 mg d'EDCI. On obtient ainsi les dérivés (hémoglobine réticulée) 10, 11 et 12.

## Tableau I

### Couplage hémoglobine (Hb) - BHC

| Exemples | Rapport molaire BHC/Hb | Rapport molaire EDCI/Hb | Hb$_4$ non modifiée % (a) | Hb$_4$ modifiée totale (e) % | Hb$_4$ réticulée % (b) | P$_{50}$ Pa (c) |
|---|---|---|---|---|---|---|
| 1 | 3 | 4 | 48 | 52 | 7 | 2120 |
| 2 | 3 | 6 | 44 | 56 | ∼ 50 | 1970 |
| 3 | 5 | 6 | 15 | 85 | 25 | 1210 |
| 4 | 3 | 8 | 20 | 80 | 80 | 1200 |
| 5 | 5 | 8 | 9 | 91 | 80 | 870 |
| 6 (d) | 3 | 8 | 12 | 88 | 68 | 2270 |

EP 0 528 716 A1

EP 0 528 716 A1

Tableau II

Couplage hémoglobine (Hb) – BTC

| Exemples | Rapport molaire BHC/Hb | Rapport molaire EDCI/Hb | $Hb_4$ non modifiée % (a) | $Hb_4$ modifiée totale (e) % | $Hb_4$ réticulée % (b) | $P_{50}$ Pa (c) |
|---|---|---|---|---|---|---|
| 7 | 5 | 8 | 17 | 83 | 13 | 2025 |
| 8 | 10 | 16 | 14 | 86 | 20 | 1865 |
| 9 | 20 | 32 | 10 | 90 | 18 | 930 |
| 10 | 20 | 24 | 18 | 82 | 8,5 | 1500 |
| 11 | 5 | 16 | 3 | 97 | 28 | 1970 |
| 12 | 5 | 32 | 3 | 97 | 61 | 1000 |

Légendes des tableaux :

(a) Pourcentage déterminé à partir des chromatogrammes sur Mono S (échange de cations)

(b) Pourcentage déterminé à partir des chromatogrammes sur Sephacryl S100 en milieu $MgCl_2$ 1M

(c) En tampon Tris 0,05 M, pH 7,2, 25°C ; la $P_{50}$ de l'hémoglobine native dans les mêmes conditions est de 500 Pa et en présence de DPG, la $P_{50}$ max est de 1330 Pa.

(d) Réaction réalisée en présence de NaCl 0,1 M.

(e) Inclut les molécules d'hémoglobine réticulées conformément à l'invention et celles auxquelles des molécules polycarboxyliques se sont fixées sans créer le pont de réticulation (par exemple fixation à l'extérieur de la protéine) ; (e) = 100 - (a).

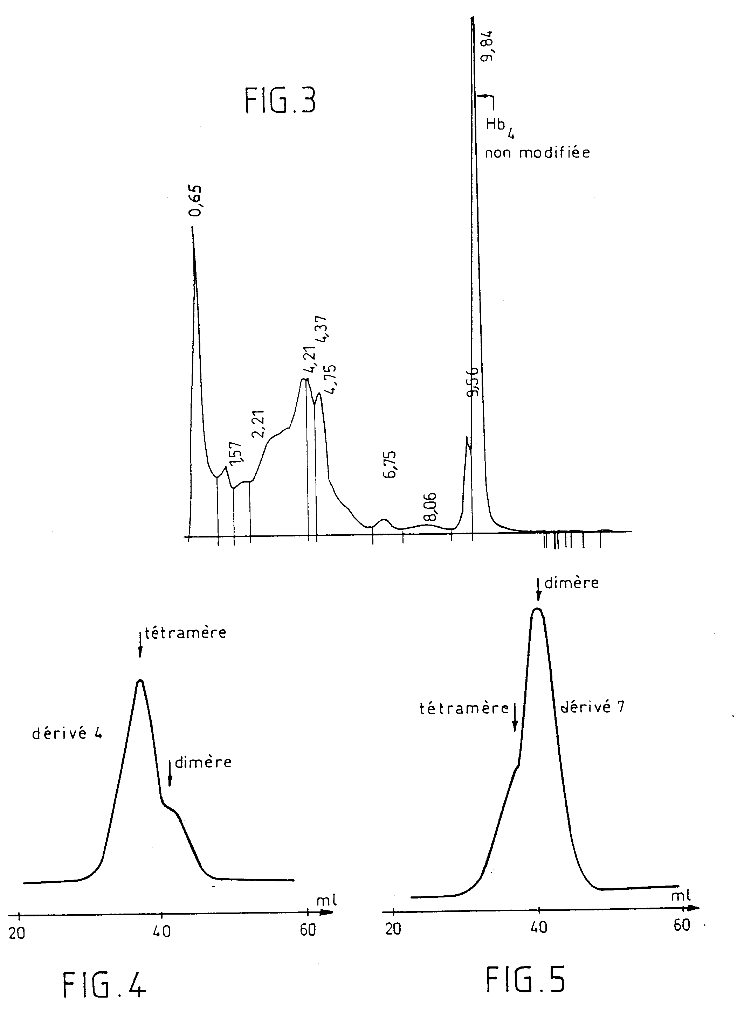

$Hb_4$ = hémoglobine sous forme tétramérique exclusivement (même en milieu dissociant tel que $MgCl_2$ que l'on rencontre dans les chromatographies sur Sephacryl S100 - figure 4).

L'analyse des exemples doit tenir compte à la fois du rendement de réticulation et de la $P_{50}$ de chaque dérivé. Une solution appropriée devrait comporter de préférence au moins 50 % environ d'hémoglobine réticulée, 80 % étant considéré comme un très bon résultat, pour un dérivé ayant une $P_{50}$ au moins égale à 1000.

La disparité des résultats obtenus pour chaque benzène polycarboxylique montre l'importance des conditions de réaction, à savoir essentiellement les rapports molaires molécule polycarboxylique/Hb et EDCI/Hb (l'EDCI favorise la création de liaisons covalentes) et, éventuellement, la présence de NaCl (exemple 6). Pour chaque molécule polycarboxylique candidate, il sera aisé à l'homme du métier de déterminer les conditions optimales.

Dans le cas présent, les exemples 4, 6 et 12, et dans une moindre mesure l'exemple 2, sont très satisfaisants.

Sur la figure 3, on observe, en se basant sur l'exemple 4, la multiplicité des espèces formées après couplage au BHC. Ces espèces diffèrent entre elles par leur acidité (molécules moins retardées sur la colonne échangeuse de cations que le tétramère initial non modifié). Dans cet exemple, le % d'hémoglobine non modifiée est de 20. La plus ou moins forte acidité des espèces modifiées dépend du nombre de résidus BHC acides fixés sur la molécule.

Sur les figures 4 et 5, on a représenté les profils obtenus en filtration sur gel en milieu $MgCl_2$ pour les exemples 4 (80 % de réticulation, $P_{50}$ = 1200) et 7 (13 % de réticulation, $P_{50}$ = 2025). Ces profils montrent très clairement les proportions respectives de dimères et de tétramères réticulés (rappelons en effet qu'en milieu $MgCl_2$, l'hémoglobine est complètement dissociée en dimères, comme le montre la figure 1).

Les dérivés 1 à 12 ont été réunis pour observer leur distribution par taille, entre eux et par rapport à l'hémoglobine native. Le chromatogramme de la figure 2 montre un pic unique se superposant à celui de l'hémoglobine native, ce qui montre que, de façon très avantageuse, il n'y a pas de réticulation intermoléculaire entre les molécules d'hémoglobine. En outre, aucun pic n'est observé au volume d'élution du BHC et du BTC.

Un autre avantage important de l'invention consiste dans le fait que le procédé s'applique à l'oxyhémoglobine, ce qui offre une grande facilité de manipulation. Bien entendu, les mêmes résultats sont obtenus avec la désoxyhémoglobine.

Si, dans les exemples 1 à 12, le couplage covalent a été réalisé en présence d'EDCI, il n'en demeure pas moins que les autres méthodes de couplage habituellement utilisées en synthèse peptidique sont également appropriées.

**Revendications**

1. Dérivé d'hémoglobine tétramérique humaine provenant du couplage covalent d'un tétramère d'hémoglobine et d'un composé polycarboxylique ayant au moins 4 groupements -COO⁻, ledit composé polycarboxylique assurant une réticulation interne des deux dimères de l'hémoglobine et lui conférant une affinité pour l'oxygène compatible avec une utilisation in vivo du conjugué hors de la présence de l'effecteur allostérique naturel de l'hémoglobine.

2. Dérivé d'hémoglobine selon la revendication 1, caractérisé en ce que les molécules polycarboxyliques ont de 4 à 6 groupements -COO⁻.

3. Dérivé d'hémoglobine selon la revendication 1 ou 2, caractérisé en ce que les molécules polycarboxyliques sont des molécules monocycliques saturées ou non saturées ou des molécules linéaires ramifiées ou non ramifiées de 4 à 6 atomes de carbone pour la chaîne principale.

4. Dérivé d'hémoglobine selon la revendication 3, caractérisé en ce que la molécule monocyclique est un benzène polycarboxylique.

5. Dérivé d'hémoglobine selon la revendication 4, caractérisé en ce que la molécule polycarboxylique est le benzène hexacarboxylique.

6. Dérivé d'hémoglobine selon la revendication 4, caractérisé en ce que la molécule polycarboxylique est le benzène tétracarboxylique.

7. Dérivé d'hémoglobine selon la revendication 3, caractérisé en ce que la molécule polycarboxylique est un cyclohexane polycarboxylique.

8. Solution de transport d'oxygène à base d'hémoglobine tétramérique humaine, dans laquelle l'hémoglobine est constituée au moins en partie par un dérivé selon l'une quelconque des revendications 1 à 7.

9. Procédé de préparation d'un dérivé d'hémoglobine tétramérique, dans lequel on effectue le couplage covalent d'une molécule d'hémoglobine tétramérique, de préférence d'oxyhémoglobine, et d'un composé polycarboxylique selon l'une quelconque des revendications 1 à 7, dans des conditions assurant la réticulation des deux dimères de la molécule d'hémoglobine.

10. Procédé selon la revendication 9, caractérisé en ce qu'on effectue le couplage en présence d'un agent favorisant les liaisons covalentes.

11. Procédé selon la revendication 10, caractérisé en ce que ledit agent est le chlorhydrate de N'-éthyl-N-(3-diméthyl-aminopropyl)carbodiimide (EDCI).

12. Procédé selon l'une quelconque des revendications 8 à 10, caractérisé en ce qu'on effectue le couplage dans des conditions assurant un rendement de réticulation d'au moins 50 % environ, notamment supérieur ou égal à 80 % environ, pour une $P_{50}$ du dérivé supérieure ou égale à 1000 Pa environ, notamment supérieure ou égale à 1300 Pa environ.

13. Procédé selon la revendication 9, caractérisé en ce que l'on effectue le couplage covalent d'hémoglobine et de benzène hexacarboxylate (BHC) avec des rapports molaires BHC/Hb et EDCI/Hb de respectivement 3 et 8 ou 5 et 6 environ.

14. Procédé selon la revendication 9, caractérisé en ce que l'on effectue le couplage covalent d'hémoglobine et de benzène tétracarboxylate (BTC) avec des rapports molaires BTC/Hb et EDCI/Hb de respectivement 5 et 32 environ.

15. Procédé selon l'une quelconque des revendications 9 à 13, caractérisé en ce qu'on effectue le couplage en présence de NaCl.

16. Solution d'hémoglobine tétramérique stabilisée obtenue par le procédé selon l'une quelconque des revendications 9 à 15.

**17.** Solution d'hémoglobine selon la revendication 16, comprenant plus de 50 %, de préférence plus de 80%, de dérivé d'hémoglobine ayant une $P_{50}$ supérieure ou égale à 1000 Pa, de préférence supérieure ou égale à 1300 Pa.

**18.** Utilisation des dérivés ou solutions selon l'une quelconque des revendications 1 à 8 et 16, 17 pour la fabrication d'une préparation de transport d'oxygène chez l'homme.

FIG.1

FIG.2

# FIG.3

# FIG.4

# FIG.5

Office européen
des brevets

## RAPPORT DE RECHERCHE EUROPEENNE

Numero de la demande

EP    92 40 2237

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | EP-A-0 338 916 (INSTITUT MERIEUX)<br>* page 7, ligne 3 - ligne 7;<br>revendications *<br>--- | 1-18 | C07K13/00<br>A61K37/14 |
| D,A | CHEMICAL ABSTRACTS, vol. 83, no. 1,<br>7 Juillet 1975, Columbus, Ohio, US;<br>abstract no. 2784w,<br>A. DESBOIS ET AL. 'EFFECTS OF POLYVALENT<br>ANION BINDING TO HEMOGLOBIN ON OXYGEN AND<br>OXIDATION-REDUCTION EQUILIBRIUMS AND THEIR<br>RELEVANCE TO ALLOSTERIC TRANSITION.'<br>page 261 ;<br>* abrégé *<br>& J. MOL. BIOL.<br>vol. 92, no. 3, 1975,<br>pages 479 - 493<br>--- | 1-18 | |
| A | EXPERIENTIA<br>vol. 35, no. 5, Mai 1979, BASEL CH<br>pages 682 - 683<br>P. LABRUDE ET AL. 'ATTEMPTS TO USE<br>CARBODIIMIDE (EDCI) TO CROSS-LINK<br>HEMOGLOBIN FOR TRANSFUSIONS.'<br>* le document en entier *<br>----- | 1-18 | **DOMAINES TECHNIQUES RECHERCHES (Int. Cl.5 )**<br><br>C07K<br>A61K |

Le présent rapport a été établi pour toutes les revendications

| Lieu de la recherche | Date d'achèvement de la recherche | Examinateur |
|---|---|---|
| LA HAYE | 11 NOVEMBRE 1992 | RYCKEBOSCH A.O. |

EPO FORM 1503 03.82 (P0402)